# EUROPEAN PATENT APPLICATION

(11) **EP 1 431 307 A1**
(43) Date of publication of application: **23.06.2004**
(21) Application number: 02028657.1
(22) Date of filing: 20.12.2002
(51) Int. Cl.: C07K 14/47, C12N 15/10, G01N 33/53, A61K 31/7088, A61K 38/00, C12N 5/06, A01K 67/027

(54) **Inactive transcription factor TIF-IA and uses thereof**

(71) Applicant: Deutsches Krebsforschungszentrum Stiftung des öffentlichen Rechts, 69120 Heidelberg (DE)
(72) Inventor: Grummt, Ingrid, Prof. Dr., 69118 Heidelberg (DE); Zhao, Jian, 69121 Heidelberg (DE)
(74) Representative: Schüssler, Andrea, Dr.

(57) **Abstract**

Described are inactive forms of the human transcription initiation factor TIF-IA, preferably a mutant form of TIF-IA which lacks functionally important posttranslational modifications, e.g. phosphorylation, acetylation, glycosylation etc. Moreover, nucleic acid molecules encoding said TIF-IA are described as well as recombinant vectors containing said nucleic acid molecules, host cells and transgenic non-human animals. Various therapeutic uses are also described which are based on the finding that ribosomal transcription depends on a properly modified TIF-IA and that by blocking, e.g. the phosphorylation of TIF-IA, cell proliferation, e.g. proliferation of cancer cells can be reduced or inhibited.

## Description

The present invention relates to inactive forms of the human transcription initiation factor TIF-IA, preferably to a mutant form of TIF-IA which lacks functionally important modifications, such as phosphorylation, acetylation or glycosylation. The present invention also relates to nucleic acid molecules encoding said TIF-IA as well as recombinant vectors containing said nucleic acid molecules, host cells and transgenic non-human animals. Furthermore, the present invention relates to various therapeutic uses based on the finding that ribosomal transcription depends on a properly modified TIF-IA and that by blocking, e.g. the phosphorylation of TIF-IA, cell proliferation, e.g. proliferation of cancer cells can be reduced or inhibited.

Regulation of cell growth and proliferation by extracellular signals is achieved by a variety of intracellular signal transduction pathways that modify and regulate the transcriptional machinery of particular target genes. Among these, the Ras-dependent mitogen-activated protein kinases (MAPKs) play a central role in transducing extracellular signals to cellular target proteins, and therefore, are of ubiquitous importance in normal as well as malignant cell growth. In this pathway, GTP-bound active Ras initiates a kinase cascade, involving sequential activation of Raf, MEK and MAPKs of the ERK (extracellular signal-regulated kinase) family. Once activated, ERK can migrate into the nucleus, phosphorylate specific regulatory proteins, and induce transcription of immediate-early genes. Moreover, ERK phosphorylates and activates RSK1-4 (90 kDa ribosomal S6 kinase1-4), a family of four closely related serine/threonine kinases. This control is exerted through phosphorylation of transcription factors like c-Fos and estrogen receptor-α, by chromatin remodeling through phosphorylation of histone H3 at Ser10 or modulation of CBP/p300 histone acetylase activity Despite the list of ERK substrates is steadily growing, the identification of key targets of ERK and RSK in mitogenic signaling remains a major challenge.

Compared to cell division, very little is known about the mechanisms that regulate cell size. Cells must grow to a minimum size before they divide. This, in turn, depends on the cell's ribosome biogenetic and hence protein synthetic capacity. Consistent with the importance of ribosome biosynthesis in growth and cell metabolism, transcription of the genes encoding rRNA is efficiently regulated according to the physiological state of the cell. Agents and environmental factors that promote cell proliferation and growth stimulate rRNA synthesis, whereas conditions that harm cellular metabolism impair RNA polymerase I (Pol I) transcription (for review, see Grummt, Progr. Nucleic Acid Res. Mol. Biol. 62 (1999), 109-154). The mechanisms that regulate Pol I transcription in response to proliferative signals are poorly understood. Recent work has demonstrated that transcription of ribosomal DNA responds to growth factor stimulation and this activation was attributed to phosphorylation of the basal Pol I transcription initiation factor UBF. On the other hand, the key player in growth-dependent control of rDNA transcription is the transcription initiation factor TIF-IA, the mammalian homologue of yeast Rrn3p. TIF-IA was initially identified as an activity that complements transcriptionally inactive extracts obtained from quiescent mouse cells. TIF-IA activity is reduced in stationary phase cells, after drug-induced inhibition of protein synthesis and deprivation of essential nutrients. The molecular mechanisms that modulate TIF-IA activity in response to cell growth are unknown. TIF-IA has been shown to interact with both Pol I and two TAF_{I}s of the TBP-containing factor TIF-IB/SL1. This finding suggests that, by interacting with DNA-bound TIF-IB/SL1, TIF-IA links the initiation-competent Pol I entity with the rDNA promoter. It can be expected that by reducing Pol-I dependent ribosomal transricption (a) the proliferation of cells can be inhibited and (b), thus, diseases can be treated or prevented which are associated with an increased cell proliferation, e.g., tumors. However, such an approach requires a more detailed knowledge of the molecular mechanisms underlying the control Pol I transcription, i.e., the mechanisms by which growth factor signalling pathways regulate ribosomal gene transcription.

Thus, the technical problem underlying the present invention is to provide means for modulating, preferably inhibiting or reducing, Pol I transcription.

The solution to said technical problem is achieved by providing the embodiments characterized in the claims. Based on the hypothesis that TIF-IA, which plays an essential role in growth-dependent control of Pol I transcription is targeted by signal transduction pathways which are important for cell proliferation, the role of TIF-IA in mitogen-induced activation of cellular rRNA synthesis was studied.

During the experiments leading to the present invention it was found that TIF-IA is a final target of ERK-dependent signaling pathways. First, in the absence of active ERK, TIF-IA is inactive. Second, treatment of growing cells with PD98059 suppresses TIF-IA activation as well as stimulation of rRNA synthesis. Third, Pol I transcription in nuclear extracts from PD98059-treated cells was restored by TIF-IA. Activation of Pol I transcription by TIF-IA was also observed if transcription was performed in the presence of the non-hydrolysable nucleotide analogues AMP-PNP and GMP-PNP. This excludes the possibility that TIF-IA-mediated transcriptional activation in extracts from PD98059-treated cells is caused by phosphorylation of UBF. Fourth, upon activation, ERK and RSK kinases associate with TIF-IA and phosphorylate serines 633 and 649, respectively. Moreover, no change of the tryptic phosphopeptide pattern of UBF was observed after estradiol treatment of cells that contain Raf-1 fused to the estrogen receptor hormone binding domain. Finally, a TIF-IA point mutant (S649A) abrogates Pol I transcription and inhibits cell growth. Thus, phosphorylation of TIF-IA by ERK-dependent pathways is a prerequisite for Pol I transcription.

TIF-IA is phosphorylated at multiple sites, the physiological relevance of most of them is not known and the respective protein kinases yet need to be identified. Using a combination of different analytical and functional assays, it could be established that the Ras-ERK pathway targets S649 and S633 within the C-terminal tail of TIF-IA. Both S649D and S633DS649D mutants exhibit a higher transcriptional activity than wild-type TIF-IA. The respective S→A mutants, on the other hand, are transcriptionally inactive and impair cell growth. Notably, S649 is not contained within a known consensus motif of the N-terminal kinase domain of RSK, raising the possibility that S649 is phosphorylated by the C-terminal kinase domain (CTK). The C-terminus of TIF-IA is phosphorylated in a hierarchical manner. Phosphorylation of S649 by RSK precedes and is obligatory for subsequent phosphorylation of S633 by ERK. Both in vivo and in vitro results demonstrate that S633 is phosphorylated in TIF-IAS649D but not of TIF-IAS649A. S633 is also in a consensus motif for p38 or JNK MAP kinases. These MAPKs, however, are normally activated by cell stress or nutrient starvation, i.e., when TIF-IA activation is not warranted. In this case, phosphorylation of S633 will preclude S649 phosphorylation by RSK and thereby TIF-IA activation. In contrast, ERK will activate RSK enabling the correct sequential phosphorylation of TIF-IA. In support of this scenario, it was found that RSK promotes the recruitment of ERK to TIF-IA. It is also of interest to note that single-residue substitution at S635 (phosphopeptide c) by aspartic acid (TIF-IAS635D) prevents in vivo phosphorylation of S633 and S649, whereas both sites are phosphorylated in TIF-IAS635A. The finding that phosphorylated S635 abrogates RSK- and ERK-mediated phosphorylation of TIF-IA suggests the existence of a network of activating and inhibiting protein kinases that modulate TIF-IA activity in vivo.

Given that TIF-IA activity is regulated by diverse signals that affect signal cell metabolism and growth, the complexity of the phosphorylation pattern of TIF-IA is not surprising. Apparently, TIF-IA is targeted by several kinase cascades and integrates multiple signaling pathways to regulate rDNA transcription either globally or in a specific fashion. The finding that the activity of TIF-IA, a key regulator of ribosomal gene transcription, is modulated by ERK and the downstream effector RSK, connects surface receptors to critical regulatory targets within the cell and link regulation of ribosome biosynthesis to cell growth and proliferation.

To summarize, the rapid increase of Pol I transcription after growth factor stimulation is due to phosphorylation of TIF-IA by ERK and RSK. Phosphorylation of TIF-IA at serine residues 633 and 649 activates TIF-IA and enhances cellular pre-rRNA synthesis. Significantly, replacement of serine 649 by alanine abolishes TIF-IA activity, impairs Pol I transcription and retards cell growth. Thus, growth factors regulate rRNA synthesis and ribosome biogenesis by ERK/RSK-mediated phosphorylation of a basal, Pol I-associated transcription initiation factor. These data reveal an as yet unknown mechanism by which MAPK pathways can control nucleolar activity, a process that is rate-limiting for growth of mammalian cells. The understanding of TIF-IA phosphorylation at a single-residue resolution enables one to develop strategies for therapy of diseases presumably associated with or due to increased cell proliferation, e.g., human cancers and may provide molecular targets for compounds designed to block cell proliferation in cancer therapy.

### Brief description of the drawings

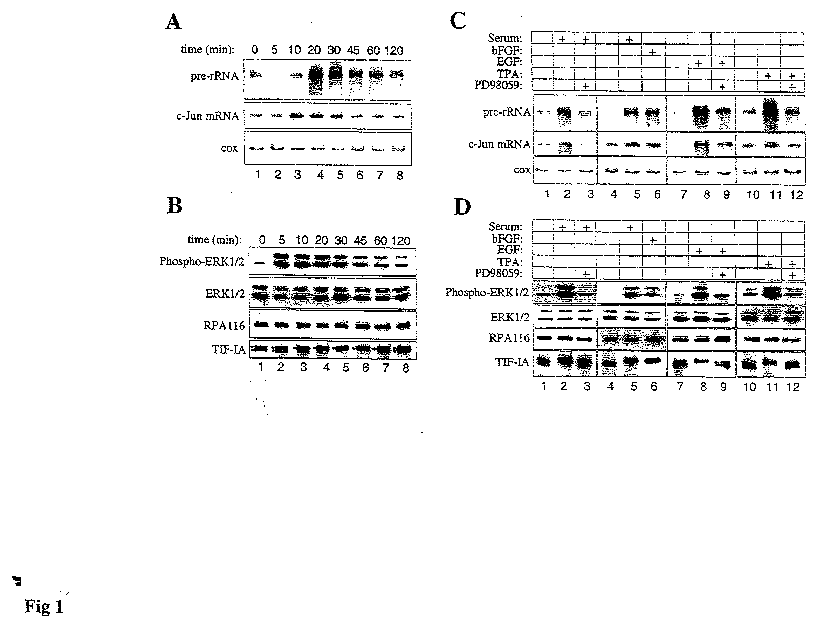

Figure 1: Cellular pre-rRNA synthesis is activated by growth factors
   **(A)** Time course of serum-induced activation of pre-rRNA synthesis. NIH3T3 cells were starved in DMEM/0.1% FCS for 12 hr before stimulation with 10% FCS. Cellular RNA was isolated at the times indicated and the relative level of pre-rRNA and c-jun mRNA was monitored on Northern blots. To normalize for variations of RNA loading, the blot was also hybridized with a probe complementary to cytochrome C oxidase (cox) mRNA.
   **(B)** Serum stimulation does not change the amount of Pol I and TIF-IA. NIH3T3 cells were starved in DMEM/0.1% FCS for 12 hr before stimulation with 10% serum. Cells were collected at the indicated times, and unphosphorylated and phosphorylated ERK1/2 as well as Pol I (RPA116) and TIF-IA were visualized on Western blots.
   **(C)** PD98059 inhibits mitogenic stimulation of Pol I transcription. NIH3T3 cells were starved in DMEM/0.1% FCS for 12 hr before stimulation by serum, 10 ng/ml bFGF, 50 ng/ml EGF or 0.1 µM TPA in the absence or presence of 40 µM PD98059 for 30 min. Cellular RNA was isolated and the level of pre-rRNA, c-jun mRNA and cytochrome C oxidase (cox) mRNA was monitored on Northern blots.
   (D) PD98059 does not affect the amount of Pol I and TIF-IA. Serum-starved NIH3T3 cells were stimulated for 30 min by 10% serum, 10 ng bFGF, 50 ng/ml EGF or 0.1 µM TPA in the absence or presence of 40 µM PD98059. A Western blot is shown with 50 µg lysate proteins and antibodies against unphosphorylated and phosphorylated ERK1/2 as well as Pol I (RPA116) and TIF-IA.

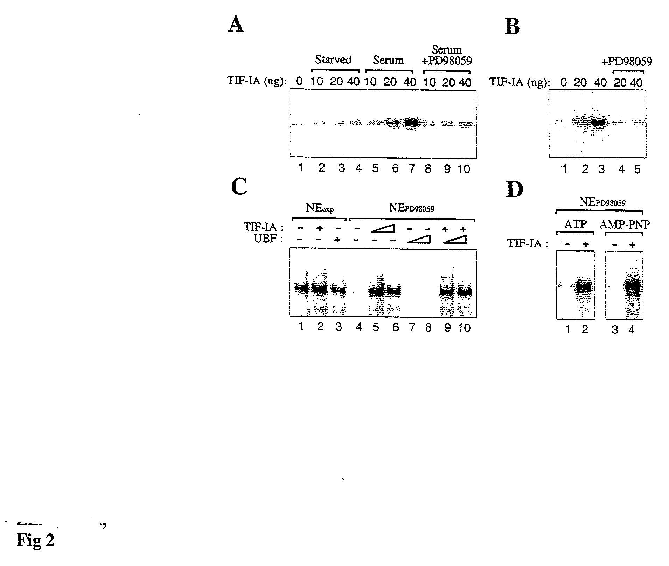

Figure 2: Inhibition of Pol I transcription in PD98059-treated cells is caused by inactivation of TIF-IA
   **(A)** TIF-IA activity in starved and serum-stimulated cells. The indicated amounts of TIF-IA purified from starved NIH3T3 cells (lanes 2-4) or cells that were stimulated by serum in the absence (lanes 5-7) or presence of PD98059 (lanes 8-10) were assayed for their capability to restore transcription of a nuclear extract from density-arrested FM3A cells (lane 1). The amount and purity of TIF-IA were estimated on silver-stained SDS-polyacrylamide gels (data not shown).
   **(B)** Inactivation of TIF-IA by inhibition of the ERK pathway. FLAG-tagged TIF-IA was purified from untreated (lanes 2-3) or PD98059-treated HEK293T cells (lanes 4-5). The indicated amounts of TIF-IA were assayed for their capability to restore transcription of a nuclear extract from density-arrested FM3A cells (lane 1). The amount of TIF-IA was estimated on silver-stained SDS-polyacrylamide gels (data not shown).
   **(C)** TIF-IA, but not UBF, rescues Pol I transcription in extracts from PD98059-treated cells. Nuclear extracts from untreated or PD98059-treated FM3A cells were supplemented with 20 and 40 ng of purified TIF-IA and/or UBF (10 ng in lanes 7 and 9, 50 ng in lanes 3, 8 and 10).
   **(D)** TIF-IA activates transcription in extracts from PD98059-treated cells in the presence of AMP-PNP and GMP-PNP. Nuclear extracts from PD98059-treated FM3A cells were supplemented with 40 ng of purified TIF-IA and assayed in the standard transcription system containing ATP and GTP (ATP, lanes 1, 2) or AMP-PNP and GMP-PNP (AMP-PNP, lanes 3, 4)

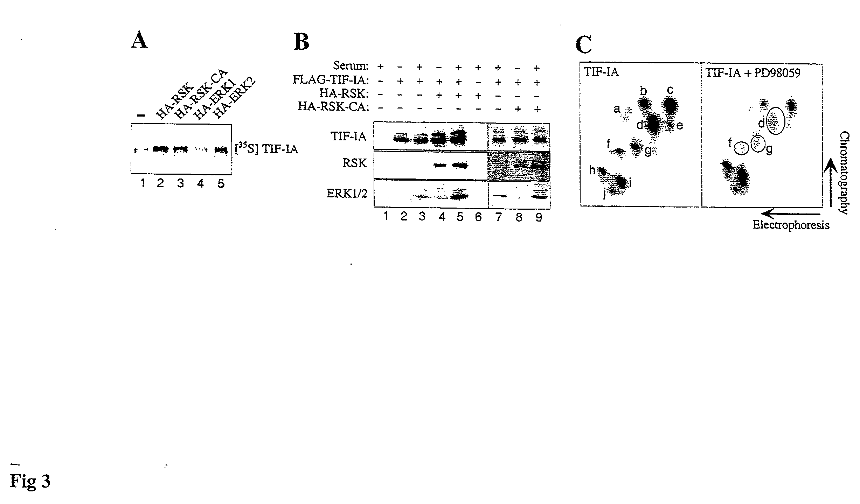

Figure 3: TIF-IA interacts with ERK and RSK
   **(A)** Pull-down assay. 6 µl of ³⁵S-labeled TIF-IA were incubated in 100 µl binding buffer with either 10 µl protein G agarose (lane 1) or bead-bound HA-RSK (lane 2), HA-RSK-CA (lane 3), HA-ERK1 (lane 4) and HA-ERK2 (lane 5). After washing and elution, bound TIF-IA was visualized by autoradiography.
   **(B)** TIF-IA interacts with ERKs and RSK. Extracts from starved or serum-stimulated HEK293T cells expressing FLAG-tagged TIF-IA and/or HA-tagged RSK2 were incubated with anti-FLAG antibodies (M2 agarose) and immunoprecipitated TIF-IA, RSK and ERK1/2 were detected on Western blots with antibodies that recognize TIF-IA, the HA-tag, or ERK1/2, respectively.
   **(C)** Two-dimensional tryptic phosphopeptide maps of TIF-IA. HEK293T cells were transfected with pcDNA3.1-FLAG-TIF-IA, starved for 12 hr and labeled for 4 hr with [³²P]orthophosphate. 30 min before harvesting, 10% dialyzed FCS was added and cells were incubated in the absence (left panel) or presence of 40 µM PD98059 (right panel). Immunopurified TIF-IA was subjected to two-dimensional phosphopeptide mapping.

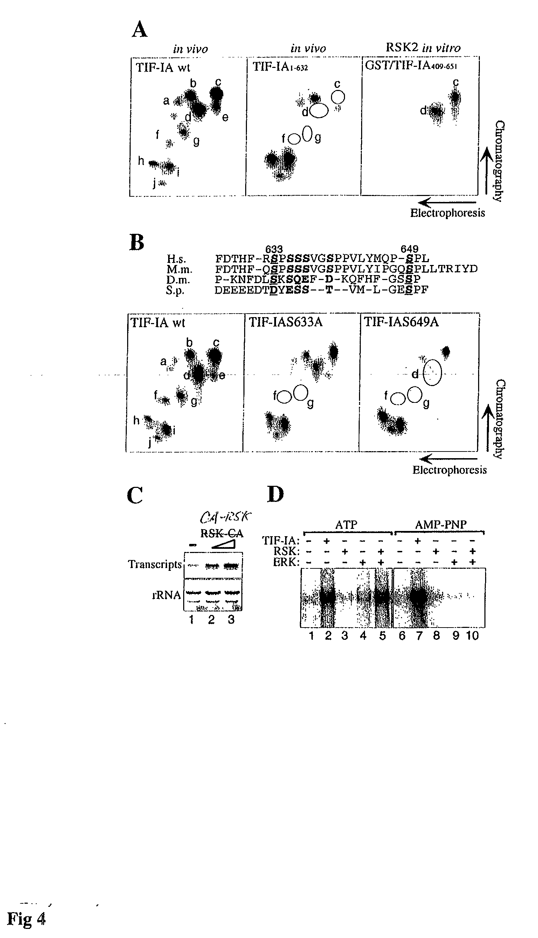

Figure 4: S633 and S649 are phosphorylated by PD98059-sensitive kinases
   **(A)** Tryptic peptide maps of recombinant TIF-IA phosphorylated by RSK2 in vitro. GST/TIF-IA₄₀₉₋₆₅₁ was phosphorylated with HA-RSK-CA in vitro and the phosphopeptide pattern was compared with that of wild-type TIF-IA and TIF-IA₁₋₆₃₂ labeled in vivo.
   **(B)** Tryptic peptide maps of TIF-IA phosphorylated in vivo. FLAG-tagged wild-type TIF-IA, TIF-IAS633A and TIF-IAS649A were expressed in HEK293T cells and labeled with [³²P]orthophosphate for 4 hr. TIF-IA was purified by immunoprecipitation and digested with trypsin. Phosphopeptides were separated on cellulose thin-layer plates and visualized by autoradiography. The sequence of the C-terminus of human TIF-IA (H.s.) and an alignment with the corresponding sequences from mouse (M.m.), *Drosophila melanogaster* (D.m.) and *Saccharomyces pombe* (S.p.) is shown above.
   **(C)** Phosphorylation by RSK is necessary and sufficient for enhancing TIF-IA activity in vivo. NIH3T3 cells were co-transfected with 3 µg pMr1930-BH and lor 2.5 µg of pMT2-HA-CA-RSK2 (lanes 2, 3). Transcripts from the reporter plasmid were monitored on Northern blots. An ethidium bromide stain of cellular 18S and 28S rRNA is shown below.
   **(D)** ERK/RSK-dependent phosphorylation activates Pol I transcription in vitro. Nuclear extracts from PD98059-treated FM3A cells were supplemented with 40 ng of TIF-IA, 10 ng of HA-RSK, or 10 ng of HA-ERK as indicated, and transcriptional activity was assayed in the standard transcription system containing ATP and GTP (lanes 4-8) or AMP-PNP and GMP-PNP (lanes 9-13).

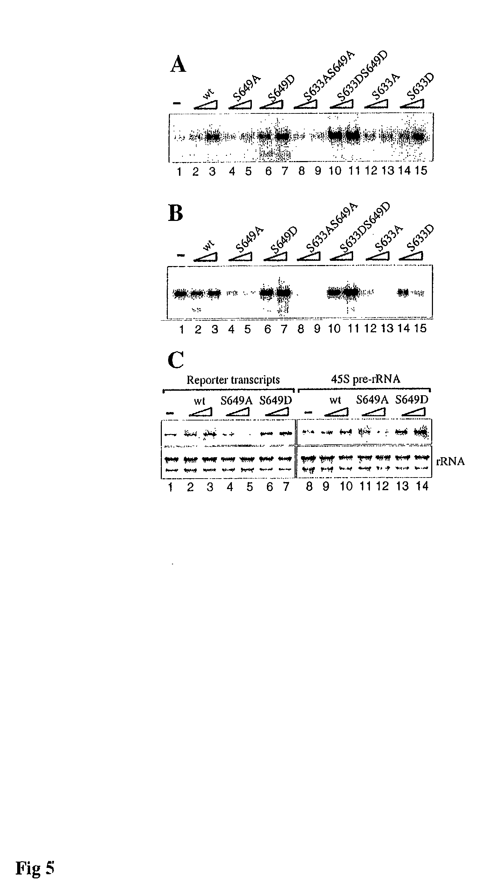

Figure 5: Phosphorylation of S649 is required for TIF-IA activity
   **(A)** Transcriptional activity of TIF-IA mutants in vitro. FLAG-tagged wild-type TIF-IA and the indicated single or double point mutants were overexpressed in HEK293T cells, immunopurified, and 20 and 40 ng were assayed for their capability to restore rDNA transcriptional activity of nuclear extract from density-arrested FM3A cells. The amount and purity of TIF-IA were estimated on silver-stained SDS-polyacrylamide gels and Western blots (data not shown).
   **(B)** TIF-IAS649A acts as a dominant-negative inhibitor of Pol I transcription. 20 and 40 ng of the indicated TIF-IA mutants were added to 15 µg nuclear extract from exponentially growing FM3A cells and rDNA transcription was assayed.
   **(C)** Overexpression of TIF-IA mutants affect cellular Pol I transcription. CLL39 cells were co-transfected with 3 µg Pol I reporter plasmid (pMr1930-BH) and 0.25 or 1 µg of pcDNA3.1-TIF-IA (lanes 2, 3) or the indicated mutants (lanes 4-7). Transcripts from the reporter plasmid were monitored on Northern blots. In lanes 8-14, HEK293T cells were transfected with 0.25 or 1 µg of the respective TIF-IA expression vectors and of cellular pre-rRNA was monitored on Northern blots. The amount of cellular 18S and 28S rRNA is shown below.

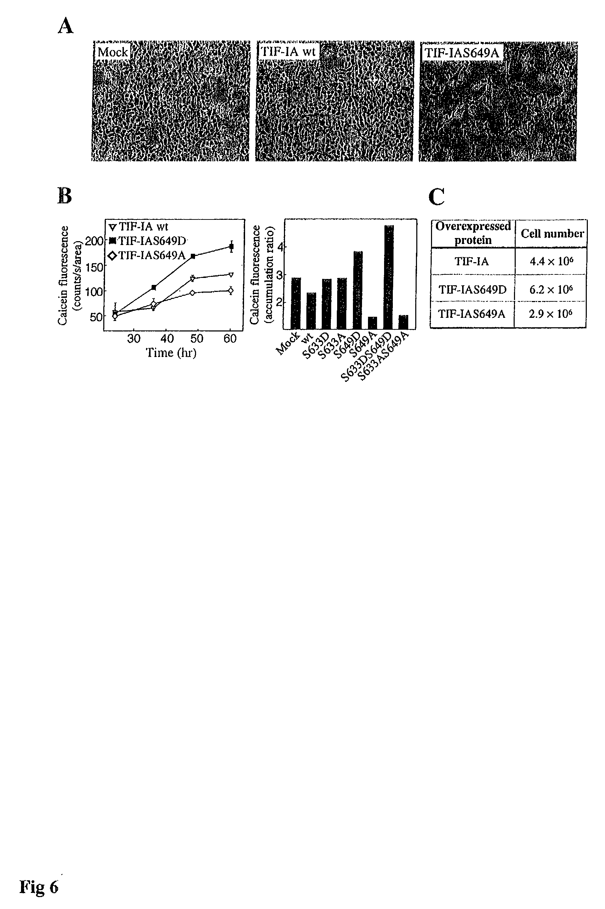

Figure 6: TIF-IAS649A inhibits cell growth
   **(A)** TIF-IAS649A retards cell growth. HEK293T cells were transfected with TIF-IA or TIFIAS649A, respectively, and images were taken after 48 h.
   **(B)** Quantitative fluorimetric viability assay with calcein-AM. HEK293T cells were transfected with TIF-IA or the indicated point mutants. Transfection efficiency proved to be >90% as determined by measuring the fluorescence of cells transfected in parallel with pcDNA-EGFP. Viable cells were measured 24, 36, 48 and 60 hr after transfection by incubation with 2 mM calcein-AM. The gradual increase in fluorescence intensity was recorded with a fluorescence multi-well plate reader for 20 min with an excitation wavelength of 485 nm and an emission wavelength of 530 nm. The proliferation rate of mock-transfected cells and cells overexpressing TIF-IA is shown at the left. Plots show the accumulation of calcein fluorescence (arbitrary units) against time. Growth rates calculated from linear regression curves are shown at the right. Values are presented as the mean ± SD from three independent experiments performed in duplicates.
   **(C)** Cell enumeration by flow cytometry. HEK293T cells expressing wild-type TIF-IA or the S649 mutants were harvested 60 hours after transfection, stained with FITC-conjugated anti-FLAG M2 antibodies and analyzed by dual-parameter flow cytometry in a FACScan™ flow cytometer in the presence of CaliBRITE™ calibration beads. 60,000 events were recorded from each sample. The number of cells expressing FLAG-TIF-IA was calculated from the ratio between FITC-positive cells and counted beads.

Thus, in a first embodiment, the present invention relates to a nucleic acid molecule encoding an inactive form of the human transcription initiation factor TIF-IA. As used herein, the term "inactive form" refers to any version of TIF-IA which has completely or partially lost its capability to initiate Pol I transcription. These versions comprise TIF-IA molecules containing substitution(s), deletion(s) and/or insertions of one or more amino acids rendering the molecule inactive and include TIF-IA which, *in vivo*, can no longer be modified in such a way that an inactive pre-form is converted into an active form. Examples of such modifications are phosphorylation, glycosylation and acetylation. The person skilled in the art can generate such versions of TIF-IA using the wild-type protein or nucleic acid (disclosed in German Patent Application 199 11 992.9-41; or available under DDBJ/EMBL/GenBank database accession no. AJ272050) as starting material. By means of conventional molecular biological processes (see, e.g., Sambrook et al., Molecular Cloning, A Laboratory Manual 2^{nd} edition (1989) Cold Spring Harbor Laboratory Press, Cold Spring Harbor, NY.) different mutations can be introduced into the wild type nucleic acid molecules. As a result, TIF-IA molecules with the desired modified biological properties are synthesized. One possibility is the production of deletion mutants in which nucleic acid molecules are produced by continuous deletions from the 5'- or 3'- terminal of the coding DNA sequence and that lead to the synthesis of polypeptides that are shortened accordingly. Another possibility is the introduction of single-point mutation(s) at positions where a modification of the amino aid sequence (e.g., glycosylation, acetylation or phosphorylation) influences particular properties. By this method versions of TIF-IA can be produced, for example, that are no longer subject to the regulatory mechanisms that normally exist in the cell, e.g. with regard to allosteric regulation or covalent modification. Such forms of TIF-IA might also be valuable as therapeutically useful antagonists of biologically active TIF-IA.

The nucleic acid molecules of the invention can be both DNA and RNA molecules. Suitable DNA molecules are, for example, genomic or cDNA molecules.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis methods usually sequence analysis, restriction analysis and other biochemical or molecular biological methods are used.

It is also to be understood that the nucleic acid molecules of the invention can be used for "gene targeting" and/or "gene replacement", or for creating a mutant gene via homologous recombination; see for example Mouellic, PNAS USA 87 (1990), 4712-4716; Joyner, Gene Targeting, A Practical Approach, Oxford University Press.

In a preferred embodiment, the nucleic acid molecule of the present invention encodes human transcription factor TIF-IA which is not or not completely posttranslationally modified, i.e., at least one amino acid within the TIF-IA amino acid sequence which is a target for modifying enzymes, e.g. proteinkinases, acetyltransferases or glycosylases has been changed (e.g., by insertion, deletion and/or substitution of amino acid(s) in such a way that it can no longer be recognized by said enzymes.

In a more preferred embodiment, the nucleic acid molecule of the present invention encodes TIF-IA, wherein the serine residue at position 633 and/or 649 of the wild type amino acid sequence is replaced by another amino acid residue which blocks phosphorylation at this site. Examples of suitable amino acid residues are alanine or glycine substitutions.

In an even more preferred embodiment, the nucleic acid molecule of the present invention encodes TIF-IA, wherein the serine residue at position 649 is replaced by an alanine residue.

The present invention furthermore relates to vectors containing the nucleic acid molecules of the invention. Preferably, they are plasmids, cosmids, viruses, bacteriophages and other vectors usually used in the field of genetic engineering. Vectors suitable for use in the present invention include, but are not limited to the CMV- and T7-based expression vector for expression in mammalian cells, preferably a vaccinia based vector, and baculovirus-derived vectors for expression in insect cells. Preferably, the nucleic acid molecule of the invention is operatively linked to the regulatory elements in the recombinant vector of the invention that guarantee the transcription and synthesis of an mRNA in prokaryotic and/or eukaryotic cells that can be translated. The nucleotide sequence to be transcribed can be operably linked to a promoter like a T7, CMV, metallothionein I or polyhedrin promoter.

In a further embodiment, the present invention relates to recombinant host cells transiently or stably containing the nucleic acid molecules or vectors of the invention. A host cell is understood to be an organism that is capable to take up *in vitro* recombinant DNA and, if the case may be, to synthesize encoded by the nucleic acid molecules of the invention. Preferably, these cells are prokaryotic or eukaryotic cells, for example mammalian cells, bacterial cells, insect cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

A further embodiment of the invention relates to an inactive human transcription initiation factor TIF-IA which is encoded by a nucleic acid molecule of the invention, as well as to methods for its production, whereby, e.g., a host cell of the invention is cultivated under conditions allowing the synthesis of inactive TIF-IA and the TIF-Ia polypeptide is subsequently isolated from the cultivated cells and/or the culture medium. Isolation and purification of the recombinantly produced TIF-IA may be carried out by conventional means including preparative chromatography and affinity and immunological separations using, e.g., an anti-TIF-IA antibody, or, e.g., can be substantially purified by the one-step method described in Smith and Johnson, Gene 67; 31-40 (1988).

The provision of the nucleic acid molecule according to the invention opens up the possibility to produce transgenic non-human animals with an inactive TIF-IA (alone or in combination with wild type, i.e. active, TIF-IA). Techniques how to achieve this are well known to the person skilled in the art. Such methods, e.g., comprise the introduction of a nucleic acid molecule or recombinant vector of the invention into a germ cell, an embryonic cell, stem cell or an egg or a cell derived therefrom.

Thus, the invention also relates to a transgenic non-human animal such as a transgenic mouse, rat, hamster, dog, monkey, rabbit, pig, C. elegans and fish such as torpedo fish comprising a nucleic acid molecule or vector of the invention, preferably wherein said nucleic acid molecule or vector is stably integrated into the genome of said non-human animal, preferably such that the presence of said nucleic acid molecule or vector leads to the expression of the inactive TIF-IA of the invention. Said animal may have one or several copies of the same or different nucleic acid molecules encoding one or several mutant forms of TIF-IA polypeptide. This animal has numerous utilities and therefore, presents a novel and valuable animal in the development of therapies, treatment, etc. for diseases caused by aberrant expression of a TIF-IA or being associated with increased cell proliferation, e.g., tumors. Accordingly, in this instance, the non-human mammal is preferably a laboratory animal such as a mouse or rat.

Preferably, the transgenic non-human animal of the invention further comprises at least one wild type allele of the corresponding TIF-IA encoding gene. This embodiment allows for example the study of the dominant negative effect of inactive TIF-IA. All the applications that have been herein before discussed with regard to a transgenic animal also apply to animals carrying two, three or more transgenes. It might be also desirable to induce inactive TIF-IA (and/or wild type TIF-IA) expression or function at a certain stage of development and/or life of the transgenic animal. This can be achieved by using, for example, tissue specific, developmental and/or cell regulated and/or inducible promoters for controlling TNF-IA expression. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62).

The non-human transgenic animals of the invention is well suited for, e.g., pharmacological studies of drugs. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe.

Since it could be shown that the inactive TIF-IA according to the present is dominant negativ, the application of TIF-IA (as protein or nucleic acid molecule encoding TF-IA) can inhibit cell proliferation. Thus, the present invention also relates to a pharmaceutical composition comprising a nucleic acid molecule, TIF-IA polypeptide or recombinant vector according to the present invention and a pharmaceutically acceptable excipient, diluent or carrier.

Examples of suitable pharmaceutical carriers etc. are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the condition or disease, general health and other drugs being administered concurrently.

The delivery of the nucleic acid molecules of the invention can be achieved by direct application or, preferably, by using a recombinant expression vector such as a chimeric virus containing these compounds or a colloidal dispersion system. Direct application to the target site can be performed, e.g., by ballistic delivery, as a colloidal dispersion system or by catheter to a site in artery. The colloidal dispersion systems which can be used for delivery of the above nucleic acid molecules include macromolecule complexes, nanocapsules, microspheres, beads and lipid-based systems including oil-in-water emulsions (mixed), micelles, liposomes and lipoplexes, The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tissues via specific cell-surface ligands.

Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo- gene therapy are described in the literature and are known to the persons skilled in the art; see, e.g., WO 94/29469 or WO 97/00957.

In order to achieve expression only in the target organ, e.g., a tumor to be treated, the nucleic acid molecules of the present invention can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

The present invention also relates to the use of the above compounds of the invention for the preparation of a pharmaceutical composition for the treatment of a disease that is caused by uncontrolled cell proliferation, e.g. cancer.

Finally, the present invention relates to a method for identifying compounds capable of inhibiting the conversion of an inactive pre-form of TIF-IA into an active form, e.g., compounds inhibiting glycosylation and/or phosphorylation of TIF-IA, said method comprising the steps of:
(a) contacting a cell which expresses TIF-IA, preferably the active wild type form, and all factors required for complete conversion of an inactive pre-form of TIF-IA into a biologically active form, preferably for glycosylation/phosphorylation of TIF-IA polypeptide, with a compound to be screened; and
(b) determining if the compound inhibits the conversion of an inactive pre-form of TIF-IA into a biologically active form, preferably glycosylation/phosphorylation of TIF-IA.

Preferably, said compound is closely related to the natural ligand or substrate of the enzymes responsible for glycosylation or phosphorylation, e.g., ERK kinases or RSK kinases, for example, a fragment of a ligand, or a structural or functional mimetic; see, e.g., Coligan, Current Protocols in Immunology 1(2) (1991); Chapter 5. The molecule can be rationally designed using known techniques.

The compounds which can be prepared and identified according to a use of the present invention may be expression libraries, e.g., cDNA expression libraries, peptides, proteins, nucleic acids, antibodies, small organic compounds, ligands, hormones, peptidomimetics, PNAs or the like. The identification of compounds which are capable of inhibiting the conversion of an inactive pre-form of TIF-IA into the active form can be performed according to methods known in the art, for example as described in EP-A 0 403 506. The compound (antagonist) identified according to the method of the invention may prove useful for therapy of diseases associated with enhanced cell proliferation, e.g., tumorous diseases.

The inhibition of conversion of the inactive form of TIF-IA into the active form, e.g., inhibition of glycosylation or phosphorylation of TIF-IA can be assayed by well known methods, e.g. the use of modification-specific antibodies.

Several methods are known to the person skilled in the art for producing and screening large libraries to identify compounds having specific affinity for a target, e.g., ERK or RSK kinases. These methods include the phage-display method in which randomized peptides are displayed from phage and screened by affinity chromatography to an immobilized receptor; see, e.g., WO 91/17271, WO 92/01047, US-A-5,223,409. In another approach, combinatorial libraries of polymers immobilized on a chip are synthesized using photolithography; see, e.g., US-A-5,143,854, WO 90/15070 and WO 92/10092. The immobilized polymers are contacted with a labeled receptor and scanned for label to identify polymers binding to the receptor. The synthesis and screening of peptide libraries on continuous cellulose membrane supports that can be used for identifying binding ligands of, e.g., ERK or RSK kinases and, thus, possible inhibitors is described, for example, in Kramer, Methods Mol. Biol. 87 (1998), 25-39. This method can also be used, for example, for determining the binding sites and the recognition motifs in a target polypeptide. Furthermore, the above-mentioned methods can be used for the construction of binding supertopes. A general route to fingerprint analyses of peptide-antibody interactions using the clustered amino acid peptide library is described in Kramer, Mol. Immunol. 32 (1995), 459-465. In addition, antagonists of enzymes like ERK and RSK kinases can be derived and identified from monoclonal antibodies that specifically react with said enzymes in accordance with the methods as described in Doring, Mol. Immunol. 31 (1994), 1059-1067.

More recently, WO 98/25146 described further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with an enzyme responsible for the generation of biologically active TIF-IA or nucleic acid molecules encoding such enzymes are, for example, the in vitro screening with the phage display system as well as filter binding assays or "real time" measuring of interaction using, for example, the BIAcore apparatus (Pharmacia).

It is also well known to the person skilled in the art, that it is possible to design, synthesize and evaluate mimetics of small organic compounds that, for example, can act as a substrate or ligand to an TIF-IA activating enzyme, e.g. an ERK or RSK kinase. For example, it has been described that D-glucose mimetics of hapalosin exhibited similar efficiency as hapalosin in antagonizing multidrug resistance assistance-associated protein in cytotoxicity; see Dinh, J. Med. Chem. 41 (1998), 981-987.

The identification of polypeptides which interact with an TIF-IA activating enzyme can also be achieved, for example, as described in Scofield (Science 274 (1996), 2063-2065) by use of the so-called yeast "two-hybrid system". In this system the target polypeptide or a smaller part thereof is linked to the DNA-binding domain of the GAL4 transcription factor. A yeast strain expressing this fusion polypeptide and comprising a lacZ reporter gene driven by an appropriate promoter, which is recognized by the GAL4 transcription factor, is transformed with a library of cDNAs which will express proteins or peptides thereof fused to an activation domain. Thus, if a peptide encoded by one of the cDNAs is able to interact with the fusion peptide comprising a peptide of an TIF-IA activating enzyme, the complex is able to direct expression of the reporter gene. In this way the nucleic acid molecules and the encoded peptide can be used to identify peptides and proteins interacting with TIF-IA activating enzymes. It is apparent to the person skilled in the art that this and similar systems may then further be exploited for the identification of inhibitors of such interaction.

Once the binding factor is identified, modulation of its binding to or regulation of expression of an TIF-IA activating enzyme can be pursued, beginning with, for example, screening for inhibitors against the binding of the binding factor to the enzyme. Repression of said enzymes could then be achieved in animals by applying the binding factor (or its inhibitor) or the gene encoding it, e.g. in an expression vector. In addition, if the active form of the binding factor is a dimer, dominant-negative mutants of the binding factor could be made in order to inhibit its activity. Furthermore, upon identification of the binding factor, further components in the pathway leading to repression of a gene involved in the activation of TIF-IA then can be identified. Modulation of the activities of these components can then be pursued, in order to develop additional drugs and methods for inhibiting the conversion of an inactive pre-form of TIF-IA into the biologically active form capable to initiate Pol I transcription. The compounds isolated by the above methods also serve as lead compounds for the development of analog compounds. Once the described compound has been identified and obtained, it is preferably provided in a therapeutically acceptable form as described above.

The following examples illustrate the invention.

### Example 1

### General methods

### (A) Plasmids

cDNAs encoding wild-type (DDBJ/EMBL/GenBank database accession No. AJ272050; German Patent Application No. 199 11 992.9-41) and mutant TIF-IA were tagged at the 5' end with sequences encoding the FLAG epitope peptide and cloned into pcDNA3.1 (Invitrogen, Groningen, Denmark). The C-terminal part of TIF-IA containing aa 410-651 was generated by PCR and inserted into pGEX-4Tl (Amersham Bioscience, Buckinghamshire, England) to yield GST/TIF-IA₄₀₉₋₆₅₁. Point mutants were constructed by overlap extension PCR using oligonucleotides that replace serines at 633, 635, 640 or 649 by alanine or aspartic acid. pMr600, the template used for in vitro transcription, contains a 622 bp PvuII fragment harboring murine rDNA sequences from -324 to +292. The reporter plasmid pMr1930-BH contains a 5'-terminal mouse rDNA fragment (from -1930 to +292) fused to a 3'-terminal rDNA fragment including two 'Sal box' terminator elements (Budde and Grummt, Oncogene *18* (1999), 1119-1124). HA-tagged murine RSK2 has been described (Zhao et al., J.Biol.Chem. *271* (1996), 29773-29779). To generate the constitutively active enzyme HA-RSK-CA, aa 376-387 of RSK2 were replaced by aa 968-980 of PRK2 (Frödin et al. EMBO J. 19 (2000) 2924-2934). In addition, T365 and S365 were replaced by glutamic acid, and the ERK docking site at the C-terminus (aa 728-740) was deleted. The resulting mutant (HA-RSK-CA) is constitutively active in a growth factor- and ERK-independent manner.

### (B) Transient transfection and RNA analysis

NIH3T3, HEK293T and CLL39 cells were cultured in Dulbecco's modified Eagle's medium (DMEM) supplemented with 10% fetal calf serum. Cells were transfected with 5 µg of DNA per 10⁶ cells using the calcium phosphate DNA coprecipitation method. Total RNA was isolated after 48 h and analyzed on Northern blots as described (Voit et al., EMBO J. *18* (1999), 1891-1899). Cellular pre-rRNA levels were monitored by hybridization to antisense RNA complementary to the first 155 nucleotides of unprocessed 45S pre-RNA. Transcripts from the rDNA reporter plasmid were monitored by hybridization to a riboprobe that is complementary to pUC sequences inserted between the rDNA promoter and terminator sequences of pMr1930-BH. To normalize for differences in RNA loading, the filter was also hybridized with a riboprobe that is complementary to cytochrome C oxidase (cox) mRNA.

### (C) In vitro transcription assays

Nuclear extracts were prepared from exponentially growing (8×10⁵ cells/ml), density-arrested or PD98059-treated (CN Bioscience, Bad Soden, Germany) 40 µM, 0.5 hr) FM3A cells (ATCC, Manassas, USA). 25 µl assays contained 50 ng of template DNA (pMr600/*Eco*RI), 30 µg nuclear extract proteins and 12 mM Hepes-KOH, [pH8.0], 0.1 mM EDTA, 5 mM MgCl₂, 80 mM KCl, 10 mM creatine phosphate, 12% (v/v) glycerol, 0.66 mM each of ATP, GTP and CTP, 0.01 mM UTP and 0.5 µCi [α-³²P]UTP (5000 Ci/mmol). After incubation for 60 min at 30 C, RNA was extracted and analyzed on non-denaturing 5% polyacrylamide gels

### (D) Purification of recombinant and cellular TIF-IA

TIF-IA was immunopurified from untransfected NIH3T3 cells or from HEK293T cells overexpressing FLAG-tagged TIF-IA. 4x10⁶ cells were lysed in 0.8 ml IP buffer (20 mM Tris-HCl [pH 7.4], 200 mM NaCl, 2 mM EDTA, 2 mM EGTA, 1% Triton X-100, 0.5 mM PMSF, 1 µg/ml aprotinin, 5 µg/ml leupeptin and 1 µg/ml pepstatin A), centrifuged at 10, 000 g for 30 min, and the supernatants were incubated for 4 hr at 4 C with either 10 µg anti-TIF-IA antibodies bound to 20 µl protein G-agarose or with anti-FLAG (M2) agarose (Sigma-Aldrich Corp., Missouri, USA). The immunocomplexes were successively washed with buffer AM (20 mM Tris-HCl [pH 8.0], 5 mM MgCl₂, 0.1 mM EDTA, 20 % glycerol) containing 400 and 100 mM KCl, respectively. The amount and purity of TIF-IA was estimated on silver-stained polyacrylamide gels and similar amounts of bead-bound TIF-IA were used in in vitro transcription assays. GST fusion proteins were expressed in *E. coli* BL21(DE3) and purified on Glutathione-Sepharose 4B according to the manufacturer's instructions.

### (E) Expression and purification of UBF

FLAG-tagged UBF1 was purified from Sf9 cells infected with recombinant baculoviruses as described (Voit et al., 1999). At 44 h post-infection, cells were lysed by sonification in buffer AM-600 containing protease inhibitors (0.5 mM PMSF; 2 µg/ml each of pepstatin, leupeptin, aprotinin) and phosphatase inhibitors (80mM β-glycerophosphate, 20 mM potassium fluoride, 1 mM sodium orthovanadate). After addition of 0.5% NP-40 and centrifugation, UBF was precipitated with bead-bound anti-FLAG antibodies. After washing with buffer AM-1000/0.5% NP-40, UBF was eluted with buffer AM-300 containing 0.1% NP-40 and 400 µg/ml FLAG-peptide.

### (F) Pull-down assays

HA-tagged ERK1&2 (Frödin et al. EMBO J. 19 (2000) 2924-2934), RSK2, and RSK2-CA were overexpressed in HEK293T cells and immunopurified with anti-HA monoclonal antibodies (12CA5) (Roche Diagnostics, Mannheim, Germany) bound to protein G agarose. 10 µl empty beads or beads containing 1 µg of ERK1, ERK2, RSK2, and RSK2-CA, respectively, were incubated with 6 µl ³⁵S-labeled TIF-IA in 100 µl binding buffer (20 mM Tris-HCl [pH 8.0], 200 mM NaCl, 2 mM EDTA, 2mM EGTA, 0.5% Triton X-100, 10 mM β-glycerophosphate, 1 mM Na₃VO₄, 5 mM K₂HPO₄) for 2 hours at 4°C. After washing with binding buffer, associated TIF-IA was separated by SDS-PAGE and visualized by autoradiography.

### (G) In vitro kinase assays

HA-tagged ERK1/2 or RSK2-CA was overexpressed in HEK293T cells, immunopurified and immobilized to protein G agarose. After stringent washing, the beads were suspended in 20 µl of 1.5×kinase assay buffer (75 mM Tris-HCl [pH 8.0], 15 mM MgCl₂, 1.5 mM DTT, 5% glycerol) and assayed for activity. 30 µl reactions contained 100 µM ATP, 0.2 µCi of [α-³²P]ATP (5000 Ci/mmol), 0.5-1 µg S6 peptide (RRSSLRA) or GST/TIF-IA (Yuan et al. EMBO Reports 3 (2002) 1082-1087) and 10 fmoles RSK. After incubation for 30 min at 30°C, 5 µl of the supernatants were spotted onto phosphocellulose paper (Whatman p81), washed with 150 mM orthophosphoric acid and quantified with a PhosphorImager (Molecular Dynamics, Inc.). Alternatively, labeled GST/TIF-IA was subjected to SDS-PAGE, blotted onto nitrocellulose, digested with trypsin and analyzed by two-dimensional phosphopeptide mapping.

### (H) Metabolic labeling and tryptic phosphopeptide analysis of TIF-IA

4x10⁶ HEK293T cells overexpressing FLAG-tagged TIF-IA were cultured in DMEM/0.1% FCS for 12 hr, then for 30 min in phosphate-free DMEM with 0.1% dialyzed FCS, before labeling with 0.5 mCi/ml [³²P]orthophosphate for 4 hr. For mitogenic stimulation, 10% dialyzed FCS was added 30 min before harvesting. Cells were lysed in modified RIPA buffer (20 mM Tris-HCl [pH 7.4], 200 mM NaCl, 2 mM EDTA, 2 mM EGTA, 1% Triton X-100, 0.1% SDS, 10 mM α-glycerophosphate, 10 mM KH₂PO₄, 1 mM Na₃VO₄, 0.5 mM PMSF, 1 µg/ml aprotinin, 5 µg/ml leupeptin, and 1 g/ml pepstatin A). TIF-IA was immunoprecipitated, separated by SDS-PAGE, transferred to nitrocellulose and visualized by autoradiography. For tryptic phosphopeptide mapping, labeled TIF-IA was digested overnight at 37 C with 5 µg trypsin (sequencing grade, Promega Corp., Madison, USA) in 50 mM ammonium bicarbonate. After lyophilisation, the peptides were resolved on cellulose thin-layer plates by electrophoresis for 25 min at 1000 V in 1% (w/v) ammonium carbonate (pH 8.9), followed by ascending chromatography in a buffer containing 62.5% isobutyric acid, 1.9% n-butanol, 4.8% pyridine and 2.9% acetic acid.

### (I) Measurements of cell viability

Conversion of calcein-AM (calcein acetoxymethyl ester, (CN Bioscience GmbH, Bad Soden, Germany) to calcein was used to measure cell proliferation. 12 hr after transfection, HEK293T cells were seeded to 96-well micro-plates at 1×10⁴ cells/well. After 24, 36, 48 and 60 hr, cells were washed with PBS, incubated with 2 µM calcein-AM, and the gradual increase in fluorescence intensity within the cells was recorded by a fluorescence multi-well plate reader (CytoFluor Series 4000/TC, PerSeptive Biosystems, (Applied Biosystems, Forster City, USA) for 20 min in 1 min intervals. Excitation wavelength used for measurements of fluorescence was 485 nm with a bandwidth of 20 nm; emission wavelength was 530 nm with a bandwidth of 25 nm. Under the experimental conditions used, there is a direct correlation between the accumulation ratio of intracellular calcein fluorescence and the number of viable cells. The data from three independent experiments were analyzed by Sigmaplot and expressed as the mean ± standard deviation. For statistical analysis, student's *t* test was used. Differences with a *P* <0.01 were considered statistically significant.

### (J) Flow cytometry

HEK293T cells were harvested 60 hr after transfection with pcDNA-FLAG-TIF-IA, fixed with 2% paraformaldehyde and permeabilized with 0.1% Triton X-100 in PBS. Transfected cells were labeled by incubation for 1 hr with 2 µg/ml FITC-conjugated anti-M2 antibodies (Sigma) and subsequently stained with 20 µg/ml propidium iodide (Sigma). Cell were analyzed with a FACSCan™ flow cytometer in the presence of CaliBRITE™ beads (Becton Dickinson, San José, USA). The number of cells expressing FLAG-TIF-IA was calculated from the ratio between FITC-positive cells and counted beads. Cell numbers determined with a CASY1 cell counter (Schärfe System, Reutlingen, Germany) were found to be in good agreement with the FACS results.

### Example 2

### Activation of ribosomal RNA synthesis after mitogenic stimulation requires activation of ERK1/2 and correlates with phosphorylation of TIF-IA

To examine the molecular mechanisms by which growth factor signaling pathways regulate ribosomal gene transcription, quiescent NIH3T3 cells were stimulated by serum and the level of pre-rRNA synthesis was monitored on Northern blots. Consistent with previous results, pre-rRNA synthesis was minimal in serum-starved cells (Figure 1A, lane 1). A more than 10-fold increase in pre-rRNA synthesis was observed 20 min after mitogenic stimulation with serum (lane 4). Activation of pre-rRNA synthesis was transient, returning to basal levels 2 hr after stimulation (lanes 5-8). The kinetics of transcriptional activation of pre-rRNA and c-jun mRNA was similar, indicating that rDNA transcription responds to growth factor stimulation like early response genes.

The rapid response of rDNA transcription to mitogenic stimulation suggests a direct signaling mechanism, possibly via the MAPK kinase cascade. Indeed, immunoblots with an antibody that recognizes phosphorylated (active) ERK1/2 revealed a marked increase in active ERK1/2 after 5 min, whereas the total amount of ERK1/2 did not change (Figure 1B). Similarly, the amount of both Pol I and TIF-IA remained the same in quiescent and serum-stimulated cells. On Western blots, TIF-IA from growing and quiescent cells appears as a closely spaced double-band (indicated by a dot and asterisk in Figure 1B) that corresponds to differently phosphorylated forms of TIF-IA. After mitogenic stimulation, the faster migrating form was converted into the more slowly migrating TIF-IA moiety. The shift in electrophoretic mobility correlated with cellular pre-rRNA synthetic activity, i.e., was predominant 20-30 min after mitogenic stimulation and returned to normal levels after 2 hr. Activation of pre-rRNA synthesis and decrease in the electrophoretic mobility of TIF-IA was also observed after stimulation of quiescent cells with growth factors, e.g. bFGF, or EGF, or with the phorbol ester TPA (Figure 1C and D), a potent stimulus of the Ras-ERK pathway, through activation of protein kinase C. Treatment with PD98059, a flavone compound that selectively inhibits MEK1/2, blocked serum- and growth factor-induced stimulation of pre-rRNA synthesis (Figure 1C) and prevented the electrophoretic mobility shift of TIF-IA (Figure 1D). The correlation between PD98059-sensitive activation of ERK1/2, retardation of the electrophoretic mobility of TIF-IA, and stimulation of cellular pre-rRNA synthetic activity suggests that activation of the Ras-ERK pathway augments TIF-IA activity.

### Example 3

### Mitogenic stimulation activates TIF-IA by an ERK-dependent mechanism

If activation of pre-rRNA synthesis was due to ERK-mediated activation of TIF-IA, then the activity of TIF-IA should be enhanced in serum-stimulated cells. To test this, TIF-IA was immunopurified both from quiescent and stimulated cells, and TIF-IA activity was assayed in vitro using nuclear extracts from density-arrested cells. Extracts from growth-inhibited cells are defective in supporting specific initiation on an rDNA promoter template, and transcriptional activity can be restored by exogenous TIF-IA. As shown in Figure 2A, TIF-IA from starved cells did not stimulate transcription (lanes 2-4), whereas the same amounts of TIF-IA from serum-stimulated cells enhanced Pol I transcription in a dose-dependent manner (lanes 5-7). This result is consistent with nutrient deprivation inactivating TIF-IA. Noteworthy, TIF-IA remained inactive if cells were stimulated with serum in the presence of PD98059 (lanes 8-10). This indicates that activation of pre-rRNA synthesis after mitogenic stimulation is brought about by ERK-mediated increase of TIF-IA activity.

To examine whether modulation of TIF-IA activity by PD98059-sensitive kinases is restricted to acutely stimulated cells or whether MAPK activity is required for TIF-IA functioning in cycling cells, unsynchronized cells were treated for 30 min with PD98059, and the activity of TIF-IA from untreated and treated cells was assayed in vitro. Clearly, TIF-IA from PD98059-treated cells was transcriptionally inactive (Figure 2B, lanes 4, 5). This result demonstrates that ERK-dependent phosphorylation is required for TIF-IA function and hence, cellular rRNA synthetic activity.

To test whether both TIF-IA and UBF, or TIF-IA alone, were targeted by ERK-mediated phosphorylation, nuclear extracts from untreated and PD98059-treated cells were assayed for rDNA transcription in the absence and presence of exogenous TIF-IA or UBF (Figure 2C). If both UBF and TIF-IA were inactivated by PD98059, rescue of Pol I transcriptional activity would require both factors, whereas TIF-IA alone would be sufficient if it was the only factor that is inactivated by inhibition of the ERK pathway. The result shown in Figure 2C demonstrates that neither exogenous TIF-IA nor UBF markedly enhanced Pol I transcription in extracts from exponentially growing cells (lanes 1-3). In contrast, transcriptional activity of extracts from PD98059-treated cells was rescued by addition of immunopurified TIF-IA from growing cells, whereas UBF had no effect (lanes 4-10). Stringently purified TIF-IA did not contain significant amounts of cellular ERK or RSK (data not shown). This excludes the possibility that stimulation by TIF-IA is caused by phosphorylation of UBF present in nuclear extracts. Consistent with this, activation of Pol I transcription by TIF-IA was also observed if transcription was performed in the presence of AMP-PNP and GMP-PNP, nucleotide analogues that cannot be used by protein kinases (Figure 2D). The fact that TIF-IA rescues Pol I transcription in conditions that do not permit de novo phosphorylation of UBF indicates that TIF-IA, but not UBF, is inactivated by inhibition of the ERK pathway.

### Example 4

### TIF-IA associates with ERK and RSK

ERK is known to associate with many of its substrates, including RSK, through non-catalytic docking sites. To determine whether ERK or RSK interact with TIF-IA, pull-down assays were performed with in vitro translated TIF-IA and immobilized ERK1, ERK2 or RSK2. TIF-IA associated with both full-length RSK2 as well as a constitutively active RSK2 mutant (HA-RSK2-CA) lacking the ERK docking site (Figure 3A, lanes 2, 3). Furthermore, TIF-IA interacted with ERK2 but not ERK1 (lanes 4, 5). To analyze the interaction of TIF-IA with ERK and RSK in vivo, co-immunoprecipitation experiments were performed. FLAG-tagged TIF-IA was overexpressed in HEK293T cells in the absence or presence of HA-RSK2, TIF-IA was precipitated and co-precipitated exogenous RSK2 and endogenous ERK1/2 were detected on Western blots. As shown in Figure 3B, a significant amount of RSK co-precipitated with TIF-IA in starved cells (lane 4). After serum-stimulation, 3-fold more RSK was associated with TIF-IA (lane 5). With regard to MAPK, ERK2 was associated with TIF-IA in serum-stimulated (lane 3) but not starved cells (lane 2), indicating that the interaction of ERK2 with TIF-IA was dependent on mitogenic stimulation. If RSK2 was overexpressed, the amount of ERK2 in the immunoprecipitates was increased (lanes 4, 5). Interestingly, the RSK2 mutant lacking the ERK docking site (HA-RSK2-CA) also increased the interaction of TIF-IA with ERK (lanes 7-9), suggesting that phosphorylation of TIF-IA by RSK enhances binding of ERK to TIF-IA. No interaction was observed between TIF-IA and the RSK-related kinase MSK1 (mitogen- and stress-activated protein kinase), which is a downstream effector of both ERK and p38 MAPKs (data not shown). Together, the results suggest that TIF-IA is targeted by ERK and RSK and the two kinases may cooperate to transduce growth signals via TIF-IA to the Pol I transcription machinery.

### Example 5

### The C-terminus of TIF-IA is phosphorylated by ERK and RSK

To identify the amino acids that are phosphorylated by ERK-dependent pathways, serum-starved cells were metabolically labeled with [³²P]orthophosphate, then stimulated with serum in the absence or presence of PD98059, and immunopurified TIF-IA was subjected to two-dimensional tryptic phosphopeptide mapping. Consistent with its role in sensing a variety of external signals, TIF-IA was phosphorylated at multiple sites. In serum-stimulated cells 10 peptides were phosphorylated (Figure 3C, left panel). In the presence of PD98059, labeling of spots **d**, **f** and **g**, was impaired (Figure 3C, right panel). In addition, phosphorylation of spots **b** and **c** was reduced. Thus, ERK-dependent kinases directly or indirectly phosphorylate TIF-IA at several sites.

In order to map the tryptic peptides that are phosphorylated by ERK and/or RSK, the phosphorylation pattern of TIF-IA was compared with TIF-IA₁₋₆₃₂, a mutant lacking the C-terminal amino acids 633-651. In TIF-IA₁₋₆₃₂, phosphopeptides **c, d, f** and **g** were absent (Figure 4A, panels 1 and 2). Three of them, e.g. peptides **d, f** and **g,** were impaired in PD98059-treated cells (Figure 3C). This result suggests that serine residues within the C-terminal tryptic peptide of TIF-IA are targeted by PD98059-sensitive kinase pathways.

Phosphoamino acid analysis has established that TIF-IA is exclusively phosphorylated on serine residues (data not shown). The C-terminal tryptic peptide of TIF-IA contains three S-P sites (Figure 4B, upper panel), a known target motif for MAPKs. Both full-length TIF-IA and GST/TIF-IA₄₀₉₋₆₅₁, were efficiently phosphorylated by immunopurified RSK2 in vitro. Two tryptic peptides were labeled that correspond to spots **c** and **d** in TIF-IA from metabolically labeled cells (Figure 4A, panel 3). Moreover, GST/TIF-IA₄₀₉₋₆₃₂ was not phosphorylated by RSK2 in vitro (data not shown), suggesting that serine(s) within amino acids 633 and 651 are targeted by RSK2.

To identify the phosphoserine residues in peptides **c**, **d**, **f**, and **g**, individual serines were mutated to alanine and the tryptic phosphopeptides of wild-type and mutant TIF-IA were compared. The two-dimensional maps in Figure 4B reveal that TIF-IAS633A lacks two phosphopeptides, i.e., spots **f** and **g**. The fact that two spots disappear after mutation of a single serine residue is most likely due to partial cleavage of the sequence F-R-(phospho)S-P behind the arginine residue. Strikingly, in TIF-IAS649A all three phosphopeptides that were sensitive to PD98059-treatment, i.e., spots **d**, **f** and **g**, were absent. This suggests that phosphorylation of serine 649 by RSK (spot **d**) is a prerequisite for phosphorylation of serine 633 by ERK (spots **f** and **g**).

To prove that phosphorylation by RSK and ERK is sufficient for mitogen-mediated enhancement of TIF-IA activity, the effect of HA-RSK-CA overexpression on transcription of the Pol I reporter plasmid (pMr1930-BH) was studied. The Northern blot in Figure 4C demonstrates that overexpression of HA-RSK-CA enhanced transcription from the ribosomal reporter gene about 7-fold. Transcriptional activation even exceeded that of overexpressing TIF-IA (data not shown). In a complementary approach, it was examined whether purified RSK and/or ERK would rescue Pol I transcription in extracts from PD98059-treated cells. Indeed, RSK plus ERK activated Pol I transcription similar as TIF-IA (Figure 4D). Activation by ERK and RSK required kinase-permissive conditions, i.e., the presence of hydrolysable purine nucleotides (lanes 1-5). If AMP-PNP and GMP-PNP were used in transcription assays, no activation was observed (lanes 6-10). These data indicate that both RSK and ERK are necessary for mitogen-mediated enhancement of TIF-IA activity.

### Example 6

### Phosphorylation of TIF-IA at S649 is essential for Pol I transcription

To assess the functional relevance of S633 and S649 phosphorylation on transcriptional activity, the respective S→A and S→D single or double point mutants were purified from transfected cells and assayed for their capability to rescue transcription of extracts from density-arrested cells. As shown in Fig 5A, mutant S649D activated Pol I transcription to a similar or even higher level compared to wild-type TIF-IA (Figure 5A, lanes 4, 5), whereas mutant S633D was less active than wild-type TIF-IA (lanes 14, 15). Significantly, TIF-IA activity was increased in the double mutant S633DS649D (lanes 8, 9), indicating that a negative charge at both positions is necessary for maximal TIF-IA activity. Replacement of serine 649 and/or 633 by alanine (S649A, S633A and S633AS649A), on the other hand, abolished TIF-IA activity (lanes 6, 7, 10-13). Thus, phosphorylation of TIF-IA at S649 and S633 is required for transcriptional activity.

Given that phosphorylation of TIF-IA at serine 649 is essential for transcription, excess of TIF-IAS649A should impair Pol I transcription. To test this, we examined the effect of exogenous wild-type and mutant TIF-IA on transcriptional activity of extracts from exponentially growing cells. Extracts from growing cells are transcriptionally active and are not stimulated by exogenous TIF-IA (Figure 5B, lanes 1-3). Both mutants S649A and S633A suppressed transcription well below the basal level (lanes 4, 5, 8, 9, and 12, 13), whereas the S→D mutation (S649D) enhanced Pol I transcription. Thus, preventing phosphorylation at S649 and 633 abrogated or significantly reduced activation of Pol I transcription in vitro, whereas replacement by an acidic amino acid stimulated transcriptional activity.

To assay transcriptional activity of these mutants in vivo, cells were co-transfected with the ribosomal reporter plasmid and increasing amounts of expression vectors harboring the respective TIF-IA S→A and S→D mutants. The effect of overexpressing wild-type and mutant TIF-IA on transcription of both the Pol I reporter plasmid and cellular rDNA synthesis is shown in Figure 5C. Consistent with the in vitro data, TIF-IAS649D activated Pol I transcription more efficiently than wild-type TIF-IA, whereas TIF-IAS649D repressed Pol I transcription in a dominant-negative manner. Thus, TIF-IA phosphorylation at serine 649 is essential for TIF-IA function and cellular pre-rRNA synthesis.

### Example 7

### Phosphorylation of TIF-IA at S649 is required for cell growth

Given the functional significance of ERK/RSK-directed TIF-IA phosphorylation on Pol I transcription, mutations that enhance or abrogate TIF-IA activity may have a positive or negative effect on cell growth. To test this, HEK293T cells were transfected with cDNAs encoding wild-type or mutant TIF-IA which harbor single or double point mutations at S649 and S633. The expression of TIF-IA was estimated on Western blots and the cellular growth rate was measured by calcein-AM staining. Calcein-AM is a non-fluorescent, hydrophobic compound that is rapidly hydrolyzed by intracellular esterases releasing hydrophilic fluorescent calcein. Thus, cellular calcein fluorescence correlates with the amount of viable cells. As shown in Figure 6, overexpression of wild-type TIF-IA did not affect cell viability and growth. Strikingly, mutants that mimic phosphorylation at residues 649 and 633 (TIF-IAS649D and TIF-IAS633DS649D) accelerated cell growth, whereas TIF-IAS649A and TIF-IAS633AS649A impaired cell growth (Figure 6B). Staining the cells with trypan blue revealed that TIF-IAS649A- and TIF-IAS633AS649D-mediated growth inhibition was not due to decreased cell viability (data not shown). To unambiguously demonstrate stimulation of growth by overexpressing TIF-IAS649D and inhibition of growth by overexpressing TIF-IAS649A, the number of cells was measured 60 hr after transfection by dual-parameter flow cytometry. Cells expressing FLAG-tagged wild-type or mutant TIF-IA were stained with FITC-conjugated anti-FLAG antibodies, identified by FACS and cell numbers were calculated by measuring the ratio of stained cells versus an internal standard. This analysis revealed that the number of cells expressing TIF-IAS649D was 43% higher compared to cells overexpressing wild-type TIF-IA (Figure 6C). Conversely, the number of cells expressing TIF-IAS649A was 33% lower than the control. The acceleration of cell growth by overexpressing TIF-IAS649D and retardation of growth by overexpressing TIF-IAS649A, respectively, suggests that the TIF-IA mutants affect cell cycle progression. Indeed, measurement of the relative DNA content of the sorted cells by FACS analysis reveals a 50% increase of G2/M-phase cells in TIF-IAS649D-expressing and a 25% decrease in TIF-IAS649A-expressing cells. Taken together, the results reveal a striking correlation between phosphorylation of TIF-IA at serine 649, cellular rRNA synthetic activity and cell cycle progression.

### Example 8

### Generation of a transgenic mouse

To study the effect of modification-deficient TIF-IA mutants on cell growth and proliferation, transgenic mice will be generated that express the desired mutants. For this, a targeting vector has been constructed that contains the cellular TIF-IA gene and a selection marker (thymidine kinase/neomycin cassette) in intron 16. Specific mutations will be introduced into defined gene regions by overlap extension PCR. Murine embryonic stem (ES) cells will be transfected with the respective targeting vectors and chimeric mice harboring one allele of mutant TIF-IA will be generated using standard protocols. Heterozygous mice generated by mating chimeric mice with strain C56B1/6 will be intercrossed to produce homozygous mice expressing mutant TIF-IA. This animal model is expected to prove the functional importance of posttranslational modifications of TIF-IA for cell growth, proliferation and differentiation.

## Claims

1. A nucleic acid molecule encoding an inactive form of the human transcription initiation factor TIF-IA.

2. The nucleic acid molecule of claim 1, wherein the human transcription initiation factor TIF-IA is not or not completely posttranslationally modified.

3. The nucleic acid molecule of claim 2, wherein the serine residue at position 633 and/or 649 is replaced by another amino acid residue.

4. The nucleic acid molecule of claim 3, wherein the serine residue at position 649 is replaced by an alanine residue.

5. A recombinant vector containing the nucleic acid molecule of any one of claims 1 to 4.

6. The recombinant vector of claim 5 wherein the nucleic acid molecule is operatively linked to regulatory elements allowing transcription and synthesis of a translatable RNA in prokaryotic and/or eukaryotic host cells.

7. The recombinant vector of claim 6 which is a vaccinia based expresssion vector.

8. A recombinant host cell which contains the recombinant vector of any one of claims 5 to 7.

9. The recombinant host cell of claim 8, which is a mammalian cell, a bacterial cell, an insect cell or a yeast cell.

10. An inactive human transcription initiation factor TIF-IA which is encoded by a nucleic acid molecule of any one of claims 1 to 4.

11. A method of producing an inactive human transcription initiation factor TIF-IA comprising:
(a) culturing the recombinant host cell of claim 8 or 9 under conditions such that said TIF-IA is expressed; and
(b) recovering said TIF-IA.

12. An inactive human transcription initiation factor TIF-IA produced by the method of claim 11.

13. A transgenic non-human animal comprising at least one nucleic acid molecule of any one of claims 1 to 4 or the recombinant vector of any one of claims 5 to 7.

14. The transgenic non-human animal of claim 13 further comprising at least one wild type allele of the TIF-IA encoding gene.

15. The transgenic non-human animal of claim 13 or 14 which is a mouse or rat.

16. A pharmaceutical composition comprising a nucleic acid molecule of any one of claims 1 to 4, a TIF-IA polypeptide of claim 10 or 12, or a recombinant vector of any one of claims 5 to 7 and a pharmaceutically acceptable excipient, diluent or carrier.

17. A method for identifying compounds capable of inhibiting the conversion of an inactive pre-form of TIF-IA into a biologically active form, said method comprising the steps of:
(a) contacting a cell which expresses TIF-IA and all factors required for said conversion of said TIF-IA with a compound to be screened; and
(b) determining if the compound inhibits the conversion of an inactive pre-form of TIF-IA into a biologically active form.

18. Use of a nucleic acid molecule of any one of claims 1 to 4, a TIF-IA polypeptide of claim 10 or 12, a recombinant vector of any one of claims 5 to 7 or a compound identified according to the method of claim 17 for the preparation of a medicament for treatment of a disease which is associated with an increased cell proliferation.

19. Use according to claim 18, wherein the disease is a tumor.
